(19) Europäisches Patentamt
European Patent Office
Office européen des brevets

(11) Publication number: **0 176 161**
**A1**

(12) # EUROPEAN PATENT APPLICATION

(21) Application number: 85301195.5

(22) Date of filing: 22.02.85

(51) Int. Cl.⁴: C 07 D 493/10
C 07 C 101/78, C 07 C 99/00
C 07 C 91/44, C 07 C 89/00
B 41 M 5/12
//(C07D493/10, 311:00, 307:00)

(30) Priority: 24.08.84 JP 177112/84
20.09.84 JP 198091/84
12.10.84 JP 214874/84
29.11.84 JP 253334/84

(43) Date of publication of application:
02.04.86 Bulletin 86/14

(84) Designated Contracting States:
CH DE FR GB LI

(71) Applicant: TAOKA CHEMICAL CO., LTD
No. 2-11, Nishimikuni 4-chome
Yodogawa-ku Osaka-shi Osaka-fu(JP)

(72) Inventor: Akamatsu, Takashi
7-1-1824, Takahama-cho
Ashiya-shi Hyogo-ken(JP)

(74) Representative: Ford, Michael Frederick et al,
MEWBURN ELLIS & CO. 2/3 Cursitor Street
London EC4A 1BQ(GB)

(54) **Novel fluoran compounds and production and use thereof.**

(57) A fluoran compound of the formula:

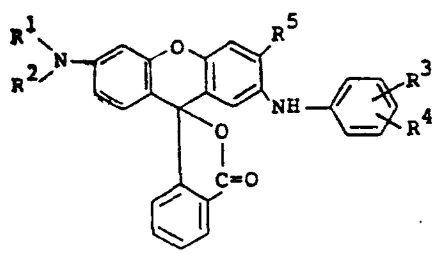

(I)

wherein $R^1$ is $C_3$-$C_{13}$ secondary alkyl with respect to the carbon atom bonded to the nitrogen atom, $R^2$ is $C_1$-$C_4$ primary alkyl, $C_3$-$C_4$ alkenyl or $C_3$-$C_4$ alkoxyalkyl, $R^3$ and $R^4$ are, same or different, hydrogen, halogen, $C_1$-$C_4$ alkyl or trifluoromethyl, $R^5$ is hydrogen, methyl, ethyl or halogen, which is useful as a color former and develops deep black with a developer.

EP 0 176 161 A1

0176161

# NOVEL FLUORAN COMPOUNDS AND PRODUCTION AND USE THEREOF

## FIELD OF THE INVENTION

The present invention relates to novel fluoran compounds and production and use thereof. More particularly, it relates to novel fluoran compounds, a method for the production of the same and a recording paper comprising the same as an electron donative color former. Further, it relates to intermediates for use in the production of the novel fluoran compounds.

## BACKGROUND OF THE INVENTION

With the development of information media, increasingly used is a pressure or heat sensitive recording paper in which color is developed by reaction between a substantially colorless electron donative compound and a substantially colorless electron accepting compound. Generally, the pressure sensitive recording paper comprises an upper sheet of paper comprising a supporting member and an electron donative white coloring matter (hereinafter referred to as a "color former") which is microcapsulated and applied thereon and a lower sheet of paper comprising a supporting member and an electron accepting compound (hereinafter referred to as a "developer") applied thereon, which sheets are laminated with their applied surface facing each other. The upper sheet may be prepared by dissolving the color former in an organic solvent, emulsifying it so that particles have a particle size of several microns, microcupsulating the emulsion with a polymeric material and applying the

microcupsulated color former on the supporting member. When pressure is applied on the pressure sensitive recording paper with a pencil or by percussion, the microcapsules are broken to liberate the color former. The liberated color former is transferred on the surface of the developer where color is developed by the reaction between the color former and the developer to record a copying image.

In general, the heat sensitive recording paper comprises finely granurated color former and developer are compounded in a binder such as a polymer (eg. polyvinyl alcohol) so that they do not contact with each other and applied on a supporting member. When a part of the paper is heated, at least one of the color former and the developer melts and contacts with another or each other whereby color is developed.

Although these recording papers are increasingly used, there is not known a single color former to develop black which satisfies all properties such as hue, self-coloring, fastness, cost, etc. Recent development of facsimile equipment which uses the heat sensitive recording paper requires high-speed recording. Therefore, a color former which can quickly develop deep black is highly desired.

Known color formers which develop black in the pressure and heat sensitive recording paper include, for example, 2-anilino-3-methyl-6-diethylaminofluoran, 2-anilino-3-methyl-6-(N-methyl-cyclohexylamino)fluoran and 2-anilino-3-methyl-6-(N-ethyl-p-toluidino)fluoran. These

fluoran compounds do not have satisfactory properties for use in preparing a recording paper for the high-speed facsimile equipment.

SUMMARY OF THE INVENTION

One object of the invention is to provide a novel fluoran compound which may be used as a color former which develops black.

Another object of the invention is to provide a method for preparing the novel fluoran compound.

Further object of the invention is to provide a color former which is colorless white crystal, heat and water resistant, and has good fastness and self coloring and can develop black when it contacts with a developer.

Yet another object of the invention is to provide a benzoylbenzoic acid derivative which is useful as an intermediate for use in the production of the novel fluoran compound.

Yet further object of the invention is to provide a recording paper comprising the novel fluoran compound.

DETAILED DESCRIPTION

According to one aspect of the invention, there is provided a novel fluoran compound of the formula:

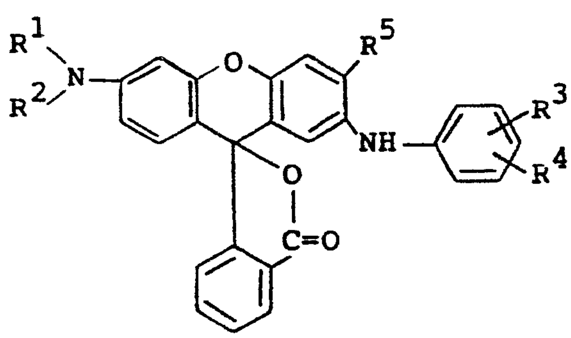

(I)

wherein $R^1$ is $C_3$-$C_{13}$ secondary alkyl with respect to the carbon atom bonded to the nitrogen atom, $R^2$ is $C_1$-$C_4$ primary alkyl, $C_3$-$C_4$ alkenyl or $C_3$-$C_4$ alkoxyalkyl, $R^3$ and $R^4$ are, same or different, hydrogen, halogen, $C_1$-$C_4$ alkyl or trifluoromethyl, $R^5$ is hydrogen, methyl, ethyl or halogen.

In the fluoran compound (I), $R^1$ is $C_3$-$C_{13}$ secondary alkyl with respect to the carbon atom bonded to the nitrogen atom. Specific examples of this substituent are 1-methylethyl, 1-methylpropyl, 1-ethylpropyl, 1-methylbutyl, 1,2-dimethylpropyl, 1,3-dimethylbutyl, 1-propylbutyl, 1-propyl-2-methylpropyl, 1-ethylpentyl, 1-methylhexyl, 1,4-dimethylpentyl, 1,3,3-trimethylbutyl, 1,3-dimethylpentyl, 1-ethyl-3-methylbutyl, 1-methylheptyl, 1-ethylhexyl, 1-ethyl-3-methylpentyl, 1,5-dimethylhexyl, 1-methyl-3-ethylpentyl, 1-ethyl-4-methylpentyl, 1-propylpentyl, 1-propyl-3-methylbutyl, 1-methyloctyl,, 1-butylpentyl, 1-isobutyl-3-methylbutyl, 1,3-dimethylheptyl, 1-ethylheptyl, 1,3-diethylpentyl, 1-butyl-3-methylbutyl, 1-methylnonyl, 1-ethyloctyl, 1-propylheptyl, 1-methyldecyl, 1-pentylhexyl, 1-methylundecyl, 1-methyldodecyl, 1-hexylheptyl, etc.

$R^2$ is $C_1$-$C_4$ primary alkyl with respect to the carbon atom bonded to the nitrogen atom, $C_3$-$C_4$ alkenyl or $C_3$-$C_4$ alkoxyalkyl. Specific examples of this substituent are methyl, ethyl, propyl, butyl, 2-methylpropyl, 2-propenyl, 2-methyl-2-propenyl, 2-methoxyethyl, 2-ethoxyethyl, etc.

$R^3$ and $R^4$ are, same or different, hydrogen, halogen such as chlorine, bromine and fluorine, $C_1$-$C_4$ alkyl such as methyl, ethyl, propyl and butyl or trifluoromethyl.

$R^5$ is hydrogen, ethyl or halogen such as chlorine, bromine and fluorine.

The novel fluoran compound (I) may be prepared by a method comprising reacting a benzoylbenzoic acid derivative of the formula:

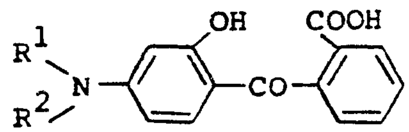

(II)

wherein $R^1$ and $R^2$ are the same as defined above with a hydroxydiphenylamine derivative of the formula:

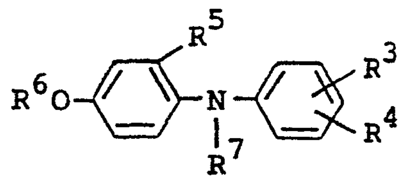

(III)

wherein $R^3$, $R^4$ and $R^5$ are the same as defined above, $R^6$ is hydrogen or $C_1$-$C_4$ alkyl and $R^7$ is hydrogen or a group of the formula: $-COR^6$ in the presence of a condensation agent at a temperature of from -5 to 90°C for several to several ten hours.

The reaction mixture is poured in water and the precipitated product is recovered, for example, by filtration and washed with water. The obtained product is heated in an aqueous alkaline solution or an organic solvent, or, in case where $R^7$ is $COR^6$, deacylated with a solution of potassium hydroxide in alcohol to obtain slightly colored

white crystal, which is filtered off, dried and recrystallized to give the white crystalline fluoran compound (I).

Specific examples of the condensation agent to be used in the above reaction of the acid derivative (II) and the derivative (III) are sulfuric acid, phosphoric acid and polyphosphoric acid. Among them, preferably 85-100 % sulfuric acid is preferred. The above condensation reaction may be carried out in a solvent such as halogenated aliphatic and aromatic hydrocarbons, acetic acid, dioxane, etc. The solvent may be removed in a suitable step of the synthesis.

After condensation, the product may be recovered by filtration. Alternatively, it may be extracted with an organic solvent which is immiscible with water which is added in the treatment with alkali.

The thus produced fluoran compound is further purified by recrystallization from an organic solvent. Specific examples of the organic solvent to be used for treatment at a high temperature or recrystallization are o-dichlorobenzene, anisole, 1,2,4-trimethylbenzene, o-xylene, isopropylbenzene, di-isopropylbenzene, paracymene, 2-ethylhexanol, cyclohexanol, benzyl alcohol, furfuryl alcohol, ethylene glycol, 2-butoxyethanol, decane, dimethylformamide, toluene, chlorobenzene, methyl isobutyl ketone, isobutanol, isopropanol, methanol, acetone, n-hexane, etc. For treatment at a high temperature, ethylene glycol and 2-butoxyethanol are preferred.

0176161

Specific example of the hydroxy diphenylamine derivative (III) are 2-methyl-4-methoxydiphenylamine, 2-ethyl-4-methoxydiphenylamine, 2,3'-dimethyl-4-ethoxydiphenylamine, 2,4'-dimethyl-4-hydroxydiphenylamine, 2-methyl-4'-chloro-4-methoxydiphenylamine, 2-chloro-4-methoxydiphenylamine, 2-chloro-4-methoxy-4'-methyldiphenylamine, 2'-chloro-4-methoxydiphenylamine, 2'-fluoro-4-methoxydiphenylamine, 4-methoxydiphenylamine, 2,2',4'-trimethyl-4-methoxydiphenylamine, 2-methyl-4-methoxy-4'-butyldiphenylamine, 2-chloro-4-methoxy-4'-butyldiphenylamine, 3'-trifluoromethyl-4-methoxydiphenylamine, etc. and their N-acyl derivatives such as N-formyl, N-acetyl, N-propanoyl and N-butanoyl derivatives.

The benzoylbenzoic acid derivative (II) is also a novel compound and within the scope of the present invention. The derivative (II) may be prepared by reacting an N-disubstituted m-aminophenol compound of the formula:

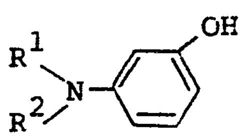

(IV)

wherein $R^1$ and $R^2$ are the same as defined above with phthalic anhydride. The compound (IV) and phthalic anhydride are reacted in a molar ratio of 1:1 to 1:2 in the absence or presence of an inert organic solvent at a temperature of from 100 to 130°C. Specific examples of the organic solvent to be used in this condensation reaction are toluene, xylene, tetrachloroethane, Perclene (Trade mark), etc.

The thus produced benzoylbenzoic acid derivative (II) may be recovered from the reaction mixture by diluting the mixture with a solvent in which the derivative is hardly soluble and filtering the derivative off, by extracting the derivative with an aqueous alkaline solution and precipitating it with acid, or by forming a sodium salt of the derivative, isolating the salt and precipitating it with acid. The derivative (II) is purified by recrystallization from an organic solvent such as toluene, isopropanol and their mixture with n-hexane.

The N-disubstituted m-aminophenol compound (IV) is a novel compound and may be prepared by reacting an N-monoalkyl-m-aminophenol compound of the formula:

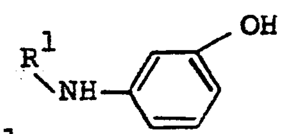

(V)

wherein $R^1$ is the same as defined above with an alkylating agent of the formula:

$$R^2-Y \quad \text{(VI)}$$

wherein $R^2$ is the same as defined above and Y is a residue of the alkylating agent such as halogen, a tosylate residue, an alkylsulfate residue, an alkylphosphate residue, an alkyl- or aryl-sulfonate residue, etc. Specific examples of the alkylating agent are methyl chloride, methyl bromide, methyl iodide, ethyl chloride, ethyl bromide, ethyl iodide, propyl chloride, propyl bromide, propyl iodide, butyl bromide, isobutyl bromide, methyl toluenesulfonate, ethyl toluenesulfonate, propyl toluenesulfonate, butyl toluenesulfonate, isobutyl toluenesulfonate, ethyl benzenesulfonate,

dimethyl sulfate, diethyl sulfate, triethyl phosphate, methoxyethyl chloride, ethoxyethyl chloride, methoxyethyl toluenesulfonate, ethoxyethyl toluenesulfonate, 2-propenyl chloride, 2-propenyl bromide, etc.

The alkylation may be carried out in the absence or presence of an solvent. Specific examples of the solvent are water, alcohols (eg. methanol, ethanol, isopropanol, etc.), ethers (eg. methoxyethanol, ethoxyethanol, ethylene glycol dimethyl ether, dioxane, etc.), dimethylformamide, dimethylsulfoxide, sulfolane, ketones (eg. acetone, methyl ethyl ketone, diethyl ketone, methyl isobutyl ketone, diisobutyl ketone, etc.), aromatic solvents (eg. toluene, xylene, chlorobenzene, etc.) and aliphatic solvents (eg. Parclene (Trademark), tetrachloroethane, etc.).

The alkylation may be carried out in the absence or presence of a deacidifier such as sodium carbonate, potassium carbonate, sodium hydrogencarbonate, sodium phosphate, sodium acetate, potassium acetate, magnesium hydroxide and calcium hydroxide.

The reaction temperature varies with a kind of the alkylating agent, and is preferably from 40 to 150°C. The reaction mixture is, if necessary, neutralized and the solvent is removed. Then, the residue was washed with water and dehydrated to obtain the produced N-disubstituted m-aminophenol (IV). The product can be purified by distillation under reduced pressure.

The N-monoalkyl-m-aminophenol (V) is a novel compound and may be prepared, for example, by following conventional three procedures:

(A) 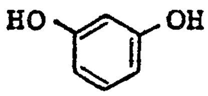 + $R^1-NH_2$ → Compound (V)

(B) 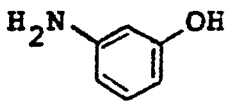 + $R^1-NH_2$ → Compound (V)

(C) 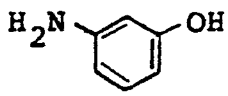 + $R^1-X$ → Compound (V)

wherein $R^1$ is the same as defined above, X is a residue of the alkylating agent such as halogen, a tosylate residue or an alkylsulfate residue.

In the procedure (A), resorcin and an amine of the formula:

$$R^1-NH_2$$

wherein $R^1$ is the same as defined above are reacted in the presence of an acidic catalyst (eg. phosphoric acid, polyphosphoric acid, phosphorous acid, toluenesulfonic acid, zinc chloride, etc.) at a temperature of from 160 to 200°C. In the procedure (B), a hydrochloride of either one of m-aminophenol and amine and the other in the free form are reacted at a temperature of from 160 to 200°C. In the procedure (C), m-aminophenol is reacted with the alkylating agent. However, in these procedures, since the reaction mixture includes not only unreacted starting materials but also by-products such as N,N'-dialkyl-m-phenylenediamine (in the procedures (A) and (B)) or N,N-dialkyl-m-aminophenol (in the procedure (C)), the isolation of N-monoalkyl-m-aminophenol requires troublesome isolation steps and yield is fairly low. In addition, in the procedure (C), when $R^1$ is a secondary alkyl, a considerable amount of olefins are formed and the yield of the compound (V) may further decreases. Therefore, the conventional procedures for producing the compound (V) are not advantageous. For the production of the compound (V) of the invention, some of the starting materials, namely, the secondary alkylamines and secondary alkylating agents are not easily available.

According to the present invention, the N-mono-secondary alkyl-m-aminophenol compound (V) is conveniently prepared by reductive alkylation, that is, by subjecting m-aminophenol to reductive alkylation with a ketone compound of the formula:

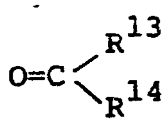

(VII)

wherein $R^{13}$ and $R^{14}$ are $R^{11}$ and $R^{12}$, respectively or groups which are converted to $R^{11}$ and $R^{12}$, respectively during the reaction, and $R^{11}$ and $R^{12}$ are, same or different, straight or branched $C_1$-$C_{11}$ alkyl provided that the total number of the carbon atoms of $R^{11}$ and $R^{12}$ is 2 to 12.

When $R^{13}$ and $R^{14}$ are $R^{11}$ and $R^{12}$ as such, respectively, specific examples of alkyl are methyl, ethyl, n-propyl, isopropyl, n-butyl, isobutyl, n-pentyl, isopentyl, neopentyl, 2-methylbutyl, n-hexyl, 2-ethylbutyl, 4-methylpentyl, n-heptyl, 2-methylhexyl, n-octyl, n-nonyl, n-decyl, n-undecyl, etc. Specific examples of the group which are converted to $R^{11}$ and $R^{12}$ are vinyl, 2-propenyl, 2-methyl-

2-hydroxypropyl, 2-methyl-2-methoxypropyl, 4-methyl-3-pentenyl, etc.

Specific examples of the ketone compound (VII) are acetone, 2-butanone, 3-pentanone, 2-pentanone, 3-methyl-2-butanone, 2-hexanone, 4-methyl-2-pentanone, 4-heptanone, 2-methyl-3-hexanone, 3-heptanone, 2-heptanone, 5-methyl-2-hexanone, 4,4-dimethyl-2-pentanone, 4-methyl-2-hexanone, 5-methyl-3-hexanone, 2-octanone, 3-octanone, 5-methyl-3-heptanone, 6-methyl-2-heptanone, 4-ethyl-2-hexanone, 6-methyl-3-heptanone, 4-octanone, 6-methyl-4-heptanone, 2-nonanone, 5-nonanone, 2,6-dimethyl-4-heptanone, 4-methyl-2-octanone, 3-nonanone, 5-ethyl-3-heptanone, 7-methyl-5-octanone, 2-decanone, 3-decanone, 4-decanone, 2-undecanone, 6-undecanone, 2-dodecanone, 2-tridecanone, 7-tridecanone, methyl vinyl ketone, diacetone alcohol, 4-methoxy-4-methyl-2-pentanone, 6-methyl-5-hepten-2-on, etc.

The reductive alkylation according to the present invention may be carried out by charging m-aminophenol, the ketone compound (VII), a solvent and a catalyst in an autoclave and reducing with hydrogen at a high temperature and high pressure. 1 Mole or more, preferably 1 to 2 mole of the ketone compound (VII) is used per mole of m-aminophenol.

Specific examples of the solvent to be used in the reductive alkylation are alcohols (eg. methanol, ethanol, isopropanol, 2-methoxyethanol, 2-ethoxyethanol, etc.), polyhydric alcohol ethers (eg. ethylene glycol dimethyl ether, diethylene glycol dimethyl ether, etc.), dioxane,

dimethylformamide, toluene, etc. The ketone compounds may serve as a solvent.

Specific examples of the catalyst for the reductive alkylation are platinum or palladium catalysts, particularly platinum carried on a carrier (eg. carbon, etc.) which is preferably slightly poisoned with a sulfur-containing compound.

The reduction is carried out with hydrogen at pressure of 5 to 60 kg/cm², preferably from 10 to 40 kg/cm² at a temperature of from 80 to 230°C, preferably from 110 to 200°C. When stoichiometric amount of hydrogen is absorbed and consumed, it is not absorbed any more and unreacted m-aminophenol disappears.

From the reaction mixture, the catalyst is removed by filtration and the solvent and excessive ketone are remove by distillation or steam distillation. Any soluble material is removed by washing with water or hot water to obtain the desired product. If desired, the product may be further purified by recrystallization or distillation under reduced pressure.

Since the reductive alkylation according to the present invention quantitatively proceeds, yield and purity of the N-monoalkyl-m-aminophenol are extremely high. Therefore, the product may be used in a subsequent reaction without isolation or purification. This is commercially advantageous.

By the reductive alkylation according to the invention, the N-monoalkyl-m-aminophenol compound (V)

wherein alkyl is $C_3$-$C_{13}$ secondary alkyl with respect to the carbon atom bonded to the nitrogen atom substantially quantitatively prepared.

According to another aspect of the present invention, there is provided a pressure or heat sensitive recording paper comprising supporting paper and at least one fluoran compound (I) and a developer applied on the paper.

The recording paper of the invention is produced by a per se conventional method. As the developer for the fluoran compound (I), bisphenols (eg. bisphenol-A, cyclohexylidene bisphenol, etc.), phenylphenol, alkylphenols, hydroxybenzoate (eg. benzyl p-hydroxybenzoate, etc.), esters of hydroxynaphthoic acid, formalin-nobolac resin of phenol or alkylphenol, condensation products of alkylphenol and sulfur chloride, zinc or calcium salt of salicylic acid or its derivatives, activated clay, clay, aromatic carboxylic acids and sulfonic acid or their metal salts, thiourea derivatives, etc.

For the production of the recording paper, any other material which is generally used in the production of a conventional recording paper such as paste, a light stabilizer and lubricant may be used. In addition, for the production of the heat sensitive recording paper, flux (a sensitivity-improving agent) is generally used. For the production of the pressure sensitive recording paper, an encapsulating material and a solvent therefor (a water-immiscible organic solvent having a high boiling point) is generally used.

The recording paper comprising the fluoran compound (I) of the invention is excellent particularly in shelf stability, coloring, light resistance, water resistance, etc. That is, when the recording paper of the invention is compared with one comprising a known color former for black, 2-anilino-3-methyl-6-(N-diethylamino)fluoran or 2-anilino-3-methyl-6-(N-cyclohexyl-N-methylamino)fluoran, in case of the pressure sensitive recording paper, the capsule-applied paper of the invention is less discolored by light, and colored image has better light and moisture resistance than the conventional one. In case of the heat sensitive recording paper, the surface of the paper and the colored image have better resistance against light, heat and moisture and greatly deep black image is developed when used in the high-speed facsimile equipment.

In Table 1 below, some fluoran compounds (I) of the invention are exemplified together with their hue, in which Hue (1) and (2) are for color developed by activated clay and bisphenol-A, respectively.

Table 1

| No. | Compound (I) | | | | | Hue (1) | Hue (2) |
|---|---|---|---|---|---|---|---|
| | $R^1$ | $R^2$ | $R^3$ | $R^4$ | $R^5$ | | |
| 1 | 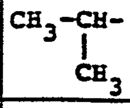 | $C_2H_5$ | H | H | $CH_3$ | Black | Black |
| 2 | 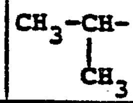 | $C_3H_7$ | H | H | $CH_3$ | Black | Black |
| 3 | 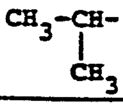 | $C_4H_9$ | H | H | $CH_3$ | Black | Black |
| 4 | 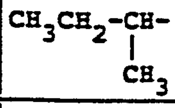 | $CH_3$ | H | H | $CH_3$ | Black | Black |
| 5 | 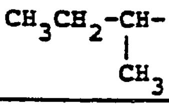 | $C_2H_5$ | H | H | $CH_3$ | Black | Black |
| 6 | 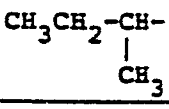 | $C_4H_9$ | H | H | $CH_3$ | Black | Black |
| 7 | 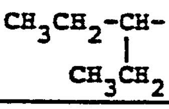 | $C_2H_5$ | H | H | $CH_3$ | Black | Black |
| 8 | 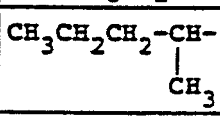 | $C_2H_5$ | H | H | $CH_3$ | Black | Black |
| 9 | 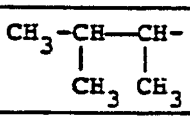 | $C_2H_5$ | H | H | $CH_3$ | Black | Black |
| 10 | 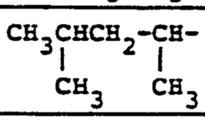 | $CH_3$ | H | H | $CH_3$ | Black | Black |
| 11 | 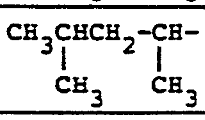 | $C_2H_5$ | H | H | $CH_3$ | Black | Black |
| 12 | 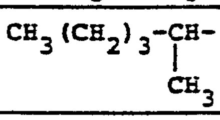 | $CH_3$ | H | H | $CH_3$ | Black | Black |
| 13 | 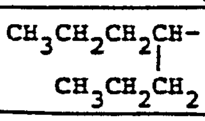 | $CH_3$ | H | H | $CH_3$ | Black | Black |
| 14 | 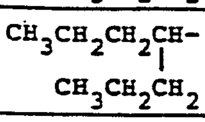 | $C_2H_5$ | H | H | $CH_3$ | Black | Black |
| 15 | 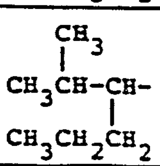 | $CH_3$ | H | H | $CH_3$ | Black | Black |
| 16 | 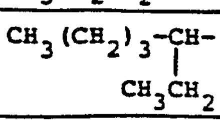 | $CH_3$ | H | H | $CH_3$ | Black | Black |

Table 1 (continued)

| No. | Compound (I) | | | | | Hue (1) | Hue (2) |
|---|---|---|---|---|---|---|---|
| | $R^1$ | $R^2$ | $R^3$ | $R^4$ | $R^5$ | | |
| 17 | $CH_3(CH_2)_4$-CH-<br>\|<br>$CH_3$ | $CH_3$ | H | H | $CH_3$ | Black | Black |
| 18 | $CH_3CH(CH_2)_2$-CH-<br>\| \|<br>$CH_3$ $CH_3$ | $C_2H_5$ | H | H | $CH_3$ | Black | Black |
| 19 | $(CH_3)_3CCH_2$CH-<br>\|<br>$CH_3$ | $C_2H_5$ | H | H | $CH_3$ | Black | Black |
| 20 | $CH_3CH_2CHCH_2$CH-<br>\| \|<br>$CH_3$ $CH_3$ | $CH_3$ | H | H | $CH_3$ | Black | Black |
| 21 | $CH_3$CH-$CH_2$-CH-<br>\| \|<br>$CH_3$ $CH_3CH_2$ | $CH_3$ | H | H | $CH_3$ | Black | Black |
| 22 | $CH_3(CH_2)_5$-CH-<br>\|<br>$CH_3$ | $CH_3$ | H | H | $CH_3$ | Black | Black |
| 23 | $CH_3(CH_2)_4$-CH-<br>\|<br>$CH_3CH_2$ | $CH_3$ | H | H | $CH_3$ | Black | Black |
| 24 | $CH_3CH_2$-CH-$CH_2$-CH-<br>\| \|<br>$CH_3$ $CH_3CH_2$ | $CH_3$ | H | H | $CH_3$ | Black | Black |
| 25 | $CH_3$CH-$(CH_2)_3$-CH-<br>\| \|<br>$CH_3$ $CH_3$ | $CH_3$ | H | H | $CH_3$ | Black | Black |
| 26 | $CH_3CH_2$-CH-$CH_2$-CH-<br>\| \|<br>$CH_3CH_2$ $CH_3$ | $CH_3$ | H | H | $CH_3$ | Black | Black |
| 27 | $CH_3$CH-$CH_2CH_2$-CH-<br>\| \|<br>$CH_3$ $CH_3CH_2$ | $CH_3$ | H | H | $CH_3$ | Black | Black |
| 28 | $CH_3CH_2CH_2$-CH-<br>$CH_3(CH_2)_3$–┘ | $CH_3$ | H | H | $CH_3$ | Black | Black |
| 29 | $CH_3$<br>\|<br>$CH_3$CH-$CH_2$-CH-<br>\|<br>$CH_3CH_2CH_2$ | $CH_3$ | H | H | $CH_3$ | Black | Black |
| 30 | $CH_3(CH_2)_6$CH-<br>\|<br>$CH_3$ | $CH_3$ | H | H | $CH_3$ | Black | Black |
| 31 | $CH_3(CH_2)_3$CH-<br>$CH_3(CH_2)_3$–┘ | $CH_3$ | H | H | $CH_3$ | Black | Black |
| 32 | $CH_3(CH_2)_3$CH-<br>$CH_3(CH_2)_3$–┘ | $C_4H_9$ | H | H | $CH_3$ | Black | Black |

Table 1 (continued)

| No. | Compound (I) | | | | | Hue (1) | Hue (2) |
|---|---|---|---|---|---|---|---|
| | $R^1$ | $R^2$ | $R^3$ | $R^4$ | $R^5$ | | |
| 33 | $(CH_3)_2CHCH_2CH-$<br>$(CH_3)_2CHCH_2$ | $CH_3$ | H | H | $CH_3$ | Black | Black |
| 34 | $(CH_3)_2CHCH_2CH-$<br>$(CH_3)_2CHCH_2$ | $C_2H_5$ | H | H | $CH_3$ | Black | Black |
| 35 | $CH_3(CH_2)_3-CHCH_2-CH-$<br>$CH_3$ $CH_3$ | $CH_3$ | H | H | $CH_3$ | Black | Black |
| 36 | $CH_3(CH_2)_5-CH-$<br>$CH_3CH_2$ | $CH_3$ | H | H | $CH_3$ | Black | Black |
| 37 | $CH_3CH_2-CH-CH_2-CH-$<br>$CH_3CH_2$ $CH_3CH_2$ | $CH_3$ | H | H | $CH_3$ | Black | Black |
| 38 | $CH_3$<br>$CH_3CHCH_2CH-$<br>$CH_3(CH_2)_3$ | $CH_3$ | H | H | $CH_3$ | Black | Black |
| 39 | $CH_3(CH_2)_7CH-$<br>$CH_3$ | $CH_3$ | H | H | $CH_3$ | Black | Black |
| 40 | $CH_3(CH_2)_6CH-$<br>$CH_3CH_2$ | $CH_3$ | H | H | $CH_3$ | Black | Black |
| 41 | $CH_3(CH_2)_5CH-$<br>$CH_3(CH_2)_2$ | $CH_3$ | H | H | $CH_3$ | Black | Black |
| 42 | $CH_3(CH_2)_8CH-$<br>$CH_3$ | $CH_3$ | H | H | $CH_3$ | Black | Black |
| 43 | $CH_3(CH_2)_4CH-$<br>$CH_3(CH_2)_4$ | $CH_3$ | H | H | $CH_3$ | Black | Black |
| 44 | $CH_3(CH_2)_4CH-$<br>$CH_3(CH_2)_4$ | $C_2H_5$ | H | H | $CH_3$ | Black | Black |
| 45 | $CH_3(CH_2)_9CH-$<br>$CH_3$ | $CH_3$ | H | H | $CH_3$ | Black | Black |
| 46 | $CH_3(CH_2)_{10}CH-$<br>$CH_3$ | $CH_3$ | H | H | $CH_3$ | Black | Black |
| 47 | $CH_3(CH_2)_5CH-$<br>$CH_3(CH_2)_5$ | $CH_3$ | H | H | $CH_3$ | Black | Black |

Table 1 (continued)

| No. | Compound (I) | | | | | Hue (1) | Hue (2) |
|---|---|---|---|---|---|---|---|
| | $R^1$ | $R^2$ | $R^3$ | $R^4$ | $R^5$ | | |
| 48 | $CH_3$-CH-<br>\|<br>$CH_3$ | $C_2H_5$ | H | 4-Cl | $CH_3$ | Purplish Black | Reddish Black |
| 49 | $CH_3$-CH-<br>\|<br>$CH_3$ | $C_2H_5$ | H | H | Cl | Black | Black |
| 50 | $CH_3$-CH-<br>\|<br>$CH_3$ | $C_2H_5$ | 2-Cl | H | H | Purplish Black | Black |
| 51 | $CH_3CH_2$CH-<br>\|<br>$CH_3$ | $C_2H_5$ | 2-Cl | H | H | Purplish Black | Black |
| 52 | $CH_3CH_2$CH-<br>\|<br>$CH_3$ | $C_2H_5$ | H | 4-Cl | $CH_3$ | Purplish Black | Reddish Black |
| 53 | $CH_3CH_2$CH-<br>\|<br>$CH_3$ | $C_2H_5$ | H | H | Cl | Black | Black |
| 54 | $CH_3CH_2$CH-<br>\|<br>$CH_3CH_2$ | $C_2H_5$ | H | H | Cl | Black | Black |
| 55 | $CH_3CH_2$CH-<br>\|<br>$CH_3CH_2$ | $C_2H_5$ | H | 4-Cl | $CH_3$ | Purplish Black | Reddish Black |
| 56 | $CH_3CH_2$CH-<br>\|<br>$CH_3CH_2$ | $C_2H_5$ | 2-Cl | H | H | Purplish Black | Black |
| 57 | $CH_3CH_2$CH-<br>\|<br>$CH_3CH_2$ | $C_2H_5$ | H | H | $C_2H_5$ | Black | Black |
| 58 | $CH_3$CH$CH_2$CH-<br>\| \|<br>$CH_3$ $CH_3$ | $C_2H_5$ | H | 4-Cl | $CH_3$ | Purplish Black | Reddish Black |
| 59 | $CH_3$CH$CH_2$CH-<br>\| \|<br>$CH_3$ $CH_3$ | $C_2H_5$ | H | H | Cl | Black | Black |
| 60 | $CH_3$CH$CH_2$CH-<br>\| \|<br>$CH_3$ $CH_3$ | $C_2H_5$ | 2-Cl | H | H | Purplish Black | Black |
| 61 | $CH_3$CH$CH_2$CH-<br>\| \|<br>$CH_3$ $CH_3$ | $C_2H_5$ | H | 3-$CH_3$ | $CH_3$ | Black | Black |
| 62 | $CH_3CH_2CH_2$CH-<br>\|<br>$CH_3CH_2CH_2$ | $CH_3$ | H | 4-Cl | $CH_3$ | Purplish Black | Reddish Black |
| 63 | $CH_3CH_2CH_2$CH-<br>\|<br>$CH_3CH_2CH_2$ | $CH_3$ | H | H | Cl | Black | Black |

Table 1 (continued)

| No. | Compound (I) | | | | | Hue (1) | Hue (2) |
|---|---|---|---|---|---|---|---|
| | $R^1$ | $R^2$ | $R^3$ | $R^4$ | $R^5$ | | |
| 64 | $CH_3CH_2CH_2CH-$<br>\|<br>$CH_3CH_2CH_2$ | $CH_3$ | 2-Cl | H | H | Purplish Black | Black |
| 65 | $CH_3CH_2CH_2CH-$<br>\|<br>$CH_3CH_2CH_2$ | $CH_3$ | 2-Cl | 4-$CH_3$ | H | Black | Black |
| 66 | $CH_3CH_2CH_2CH-$<br>\|<br>$CH_3CH_2CH_2$ | $CH_3$ | H | 4-$CH_3$ | $CH_3$ | Black | Greenish Black |
| 67 | $(CH_3)_2CHCH_2CH-$<br>\|<br>$(CH_3)_2CHCH_2$ | $CH_3$ | H | 4-$CH_3$ | $C_2H_5$ | Black | Black |
| 68 | $(CH_3)_2CHCH_2CH-$<br>\|<br>$(CH_3)_2CHCH_2$ | $CH_3$ | H | 4-Cl | $CH_3$ | Purplish Black | Reddish Black |
| 69 | $(CH_3)_2CHCH_2CH-$<br>\|<br>$(CH_3)_2CHCH_2$ | $CH_3$ | H | 4-$CH_3$ | $CH_3$ | Black | Greenish Black |
| 70 | $(CH_3)_2CHCH_2CH-$<br>\|<br>$(CH_3)_2CHCH_2$ | $CH_3$ | H | 3-$CH_3$ | $CH_3$ | Black | Black |
| 71 | $(CH_3)_2CHCH_2CH-$<br>\|<br>$(CH_3)_2CHCH_2$ | $CH_3$ | H | 3-$CF_3$ | H | Black | Black |
| 72 | $(CH_3)_2CHCH_2CH-$<br>\|<br>$(CH_3)_2CHCH_2$ | $CH_3$ | H | H | Cl | Black | Black |
| 73 | $(CH_3)_2CHCH_2CH-$<br>\|<br>$(CH_3)_2CHCH_2$ | $CH_3$ | H | H | Br | Black | Black |
| 74 | $(CH_3)_2CHCH_2CH-$<br>\|<br>$(CH_3)_2CHCH_2$ | $CH_3$ | H | 4-$CH_3$ | Cl | Greenish Black | Greenish Black |
| 75 | $(CH_3)_2CHCH_2CH-$<br>\|<br>$(CH_3)_2CHCH_2$ | $CH_3$ | 2-Cl | H | H | Purplish Black | Black |
| 76 | $(CH_3)_2CHCH_2CH-$<br>\|<br>$(CH_3)_2CHCH_2$ | $CH_3$ | H | H | H | Dark Green | Dark Green |
| 77 | $CH_3-CH-$<br>\|<br>$CH_3$ | $CH_2=CHCH_2-$ | H | H | $CH_3$ | Black | Black |
| 78 | $CH_3-CH-$<br>\|<br>$CH_3$ | $CH_3OCH_2CH_2-$ | H | H | $CH_3$ | Black | Black |
| 79 | $CH_3CH_2CH-$<br>\|<br>$CH_3$ | $CH_2=CHCH_2-$ | H | H | $CH_3$ | Black | Black |

Table 1 (continued)

| No. | Compound (I) | | | | | Hue (1) | Hue (2) |
|---|---|---|---|---|---|---|---|
| | $R^1$ | $R^2$ | $R^3$ | $R^4$ | $R^5$ | | |
| 80 | $CH_3CH_2CH(CH_3)-$ | $CH_3OCH_2CH_2-$ | H | H | $CH_3$ | Black | Black |
| 81 | $CH_3CH_2CH(CH_3)-$ | $C_2H_5$ | 2-F | H | $CH_3$ | Purplish Black | Black |
| 82 | $CH_3CH_2CH(CH_3)-$ | $C_4H_9$ | 2-F | H | $CH_3$ | Purplish Black | Black |
| 83 | $CH_3CH_2CH_2CH(CH_3)-$ | $CH_3$ | H | H | $CH_3$ | Purplish Black | Black |

The present invention will be explained further in detail by following Examples, in which parts are by weight unless otherwise indicated.

Example 1

In a 500 ml autoclave equipped with a stirrer, added were ethanol (100 g), acetone (48.8 g), m-aminophenol (76.4 g) and 5 % platinum/carbon catalyst (2 g). After replacing the interior space of the autoclave with hydrogen, the mixture was heated to 120°C and subjected to reductive alkylation with hydrogen at 20 $kg/cm^2$. As hydrogen was consumed, it was injected several times. Finally, 1 mole of hydrogen per mole of m-aminophenol was consumed. After cooling to 60°C, the reaction mixture was filtered to remove the catalyst and washed with a small amount of ethanol. Then, a great portion of ethanol was distilled off. To the residue, water (800 ml) was added and stirred to obtain a crystalline product, which was filtered, washed with water and dried to give white crystalline N-1-methylethyl-m-aminophenol (104.8 g). M.P. 99-100°C. LC purity 98.5 %. The crystalline product was recrystallized from toluene to give the white crystalline product. M.P. 100.5-101.5°C.

Chemical structure of the product was identified by elemental analysis and mass spectrum.

Example 2

In the same manner as in Example 1 but using ethanol (40 g), diacetone alcohol (25.6 g), m-aminophenol (21.8 g), glacial acetic acid (1 g) and 5 % platinum/carbon catalyst (0.8 g), the reaction, post-treatment and

recrystallization were carried out to give N-1-methylethyl-m-aminophenol (25 g). M.P. 100.5-101.5°C.

Chemical structure of the product was identified by FD mass spectrum and NMR spectrum.

Example 3

In the same manner as in Example 2 but using diacetone alcohol methyl ether in place of diacetone alcohol, the reaction and post-treatment were carried out to give N-1-methylethyl-m-aminophenol (26 g). M.P. 100.5-101.5°C.

Example 4

In the same manner as in Example 1 but using methanol (100 g), 2-butanone (60.6 g), m-aminophenol (76.4 g) and 5 % platinum/carbon catalyst (2 g), reductive alkylation was carried out with hydrogen at 120°C, and the reaction mixture was filtered to remove the catalyst. After distilling methanol off, the residue was dried to give a viscous pale yellow liquid (114 g), which was distilled under reduced pressure to give viscous pale yellow liquid N-1-methylpropyl-m-aminophenol (110 g). B.P. 154-156°C/9.5 mmHg.

Example 5

In the same manner as in Example 1 but using methanol (50 g), 3-pentanone (69.3 g), m-aminophenol (76.4 g) and 5 % platinum/carbon catalyst (1 g), reductive alkylation was carried out with hydrogen at 120°C, and the reaction mixture was filtered to remove the catalyst. After distilling methanol off, the residue was mixed with water

(800 ml). An oily product was separated and dehydrated to give a viscous pale yellow liquid (125 g), which was distilled under reduced pressure to give N-1-ethylpropyl-m-aminophenol (117 g). B.P. 164-166°C/10.5 mmHg. The liquid product was kept standing to give the white crystalline product. M.P. 40-41°C. LC purity 99.3 %.

Example 6

In the same manner as in Example 1 but using methanol (100 g), 2-pentanone (72.4 g), m-aminophenol (76.4 g) and 5 % platinum/carbon catalyst (1 g), reductive alkylation was carried out with hydrogen at 120°C, and the reaction mixture was filtered to remove the catalyst. After distilling methanol off, the residue was mixed with water (800 ml). An oily product was separated and dehydrated to give an oil (123.2 g), which was distilled under reduced pressure to give viscous pale yellow liquid N-1-methylbutyl-m-aminophenol (117 g). B.P. 162-164°C/10 mmHg. LC purity 98.9 %.

Example 7

In the same manner as in Example 6 but using 3-methyl-2-butanone (72.4 g) in place of 2-pentanone, reductive alkylation was carried out. After removing the catalyst and methanol, the reaction mixture was washed with water and dried to give white crystalline N-1,2-dimethylpropyl-m-aminophenol (120.2 g). M.P. 65.5-67°C. LC purity 98.5 %. The product was recrystallized from a mixed solvent of toluene and n-hexane to give white crystal. M.P. 67-68°C.

Example 8

In the same manner as in Example 6 but using methanol (100 g), 4-methyl-2-pentanone (84.1 g), m-aminophenol (76.4 g) and 5 % platinum/carbon catalyst (1 g), the reaction and post-treatment were carried out to give an oily product (126 g), which was distilled under reduced pressure to give viscous pale yellow liquid N-1,3-dimethylbutyl-m-aminophenol (121 g). B.P. 143-147°C/4 mmHg.

Example 9

In the same manner as in Example 1 but using methanol (100 g), 4-heptanone (68.5 g), m-aminophenol (54.6 g) and 5 % platinum/carbon catalyst (1 g), reductive alkylation was carried out with hydrogen at 180°C. After filtering the catalyst off and distilling methanol off, the reaction mixture was steam distilled to give an oily product (126 g), which was separated, washed with water, dried and then distilled under reduced pressure to give viscous pale yellow liquid N-1-propylbutyl-m-aminophenol (95 g). B.P. 147-151°C/4 mmHg.

Example 10

In the same manner as in Example 9 but using methanol (100 g), 2,6-dimethyl-4-heptanone (85.4 g), m-aminophenol (54.6 g) and 5 % platinum/carbon catalyst (1 g), the reaction and post-treatment were carried out. The oily product was dried and distilled under reduced pressure to give viscous pale yellow liquid N-1-isobutyl-3-methylbutyl-m-aminophenol (110 g). B.P. 155-158°C/4 mmHg.

Example 11

A mixture of N-1-methylethyl-m-aminophenol (15.1 g), toluene (50 g) and ethyl p-toluenesulfonate (24 g) was heated at a temperature of from 110 to 115°C for 9 hours and together with sodium acetate (9.8 g) for another 1 (one) hour. To the thus produced solution of N-ethyl-1'-methylethyl-m-aminophenol, phthalic anhydride (16.3 g) was added and heated at a temperature of from 110 to 115°C for 6 hours. Thereafter, an aqueous alkaline solution was added to the reaction mixture and stirred. After kept standing, an aqueous layer was separated and an organic layer was again washed with the aqueous alkaline solution. The combined aqueous solution was treated with active carbon and precipitated with dilute sulfuric acid. Precipitate was filtered, washed with water and dried to give 2-(4-N-ethyl-N-1'-methylethylamino-2-hydroxybenzoyl)benzoic acid (24.5 g). M.P. 165-167°C. The product was recrystallized from toluene to give white crystal. M.P. 172.5-173.5°C.

Examples 12 to 16

In the same manner as in Example 11 but using a N-monosubstituted m-aminophenol compound (0.1 mole) shown in Table 2 in place of N-1-methylethyl-m-aminophenol, the reactions were carried out to give a benzoylbenzoic acid derivative as shown in the same table.

Table 2

| Ex. No. | N-monosubstituted m-aminophenol | Benzoylbenzoic acid derivative |
|---|---|---|
| 12 | N-1-methylpropyl-m-aminophenol B.P. 154-156°C /9.5 mmHg | 2-(4-N-ethyl-N-1'-methylpropyl-amino-2-hydroxybenzoyl)benzoic acid M.P. 164.5-165.5°C*1) |
| 13 | N-1-ethylpropyl-m-aminophenol B.P. 164-166°C /10.5 mmHg M.P. 40-41°C | 2-(4-N-ethyl-N-1'-ethylpropyl-amino-2-hydroxybenzoyl)benzoic acid M.P. 175-176°C*1) |
| 14 | N-1-methylbutyl-m-aminophenol B.P. 162-164°C /10 mmHg | 2-(4-N-ethyl-N-1'-methylbutyl-amino-2-hydroxybenzoyl)benzoic acid M.P. 134-135°C*2) |
| 15 | N-1,2-dimethyl-propyl-m-amino-phenol M.P. 65.5-67°C | 2-(4-N-ethyl-N-1',2'-dimethyl-propylamino-2-hydroxybenzoyl)-benzoic acid M.P. 172.5-173.5°C*1) |
| 16 | N-1,3-dimethyl-butyl-m-amino-phenol B.P. 143-147°C /4 mmHg | 2-(4-N-ethyl-N-1',3'-dimethyl-butylamino-2-hydroxybenzoyl)-benzoic acid M.P. 141-142°C*2) |

Note: *1) Recrystallized from toluene.
*2) Recrystallized from toluene/n-hexane.

Example 17

A mixture of N-1-methylproyl-m-aminophenol (16.5 g), toluene (50 g) and methyl p-toluenesulfonate (22.3 g) was heated at a temperature of from 110 to 115°C for 8 hours and together with sodium acetate (9.8 g) for another 1 (one) hour. To the thus produced solution of N-methyl-1'-methylpropyl-m-aminophenol, phthalic anhydride (16.3 g) was added and heated at a temperature of from 110 to 115°C for 7 hours. Thereafter, the reaction mixture was treated in the same manner as in Example 11 to give 2-(4-N-methyl-N-1'-methylpropylamino-2-hydroxybenzoyl)benzoic acid (22.8 g). M.P. 163-165°C. The product was recrystallized from toluene to give white crystal. M.P. 165-166°C.

Examples 18 and 19

In the same manner as in Example 17 but using a N-monosubstituted m-aminophenol compound (0.1 mole) shown in Table 3 in place of N-1-methylpropyl-m-aminophenol, the reactions were carried out and the product was separated as a sodium salt, purified and precipitated with an acid to give a benzoylbenzoic acid derivative as shown in the same table.

Table 3

| Ex. No. | N-monosubstituted m-aminophenol | Benzoylbenzoic acid derivative |
|---|---|---|
| 18 | N-1-propylbutyl-m-aminophenol B.P. 147-151°C /4 mmHg | 2-(4-N-methyl-N-1'-propylbutyl-amino-2-hydroxybenzoyl)benzoic acid M.P. 144.5-146°C |
| 19 | N-1-isobutyl-3-methylbutyl-m-aminophenol B.P. 155-158°C /4 mmHg | 2-(4-N-methyl-N-1'-isobutyl-3'-methylbutylamino-2-hydroxybenzoyl)benzoic acid M.P. 171-172°C |

Example 20

In the same manner as in Example 11 but using n-propyl p-toluenesulfonate (26.8 g) in place of ethyl p-toluenesulfonate, the reaction was carried out to give 2-(4-N-propyl-N-1'-methylethylamino-2-hydroxybenzoyl)benzoic acid (17.7 g). M.P. 164-165°C (after recrystallization from toluene).

Example 21

In the same manner as in Example 11 but using 2-methoxyethyl p-toluenesulfonate (27.6 g) in place of ethyl p-toluenesulfonate, the reaction was carried out to give 2-(4-N-2'-methoxyethyl-N-1'-methylethylamino-2-hydroxybenzoyl)benzoic acid (24.4 g). M.P. 105-108°C.

Example 22

A mixture of N-1-methylethyl-m-aminophenol (15.1 g), toluene (50 g), allyl bromide (14.5 g), sodium iodide (0.75 g) and sodium acetate (9 g) was heated at 75°C for 1 hour and at 85°C for 2 hours. Then, the temperature was raised to 105°C over 3 hours. To the thus produced solution of N-allyl-1'-methylethyl-m-aminophenol, phthalic anhydride (16.3 g) was added and heated at a temperature of from 110 to 115°C for 8 hours. Thereafter, the reaction mixture was treated in the same manner as in Example 11 to give 2-(4-N-allyl-N-1'-methylethylamino-2-hydroxybenzoyl)benzoic acid (17.6 g). M.P. 137-139°C (after recrystallization from a mixed solvent of toluene and n-hexane).

Example 23

In the same manner as in Example 22 but using N-1-isobutyl-3-methylbutyl-m-aminophenol (23.5 g) in place of N-1-methylethyl-m-aminophenol, the reaction was carried out. The product was purified as a sodium salt and precipitated with an acid to give 2-(4-N-allyl-N-1'-isobutyl-3'-methylbutylamino-2-hydroxybenzoyl)benzoic acid. M.P. 108-110°C.

Example 24

In a 500 ml autoclave equipped with a stirrer, added were methanol (100 g), methyl ethyl ketone (60.6 g), m-aminophenol (76.4 g) and 5 % platinum/carbon catalyst (2 g). After replacing the interior space of the autoclave with hydrogen, the mixture was heated to 120°C and subjected to reductive alkylation with hydrogen at 20 kg/cm². As hydrogen was consumed, it was injected several times. Finally, 1 mole of hydrogen per mole of m-aminophenol was consumed and pure N-1-methylpropyl-m-aminophenol was produced. To the cooled autoclave, added were sodium acetate (68.9 g), potassium iodide (5.8 g) and ethyl bromide (106.8 g) and reacted at 130°C for 4 hours. After cooling, the reaction mixture was filtered and washed with methanol. The filtrate was distilled to remove materials having low boiling points including methanol, water and acetic acid to give oily N-ethyl-N-1'-methylpropyl-m-aminophenol. The thus obtained product was mixed with toluene (230 g) and phthalic anhydride (114 g) and heated at 110°C for 7 hours with stirring. The reaction mixture was extracted with an aqueous alkaline solution. An aqueous layer was separated and an organic layer was again extracted with the same aqueous solution. The combine aqueous layer was treated with active carbon and precipitated with dilute sulfuric acid. Precipitate was filtered, washed with water and dried to give 2-(4-N-ethyl-N-1'-methylpropylamino-2-hydroxybenzoyl)benzoic acid (168 g). M.P. 163-164°C. The product was recrystallized from toluene to give white crystal. M.P. 164.5-165.5°C.

Example 25

In a solution of 2-(4-N-ethyl-N-1'-methylethylamino-2-hydroxybenzoyl)benzoic acid (1.64 g. M.P. 172.5-173.5°C) in 96 % sulfuric acid (15 g), 2-methyl-4-methoxy-diphenylamine (1.07 g) was dissolved at a temperature of from 10 to 15°C and stirred at the same temperature for 24 hours. The resultant solution was poured in ice-water (80 g), and precipitate was filtered and washed with water. The filter cake was taken in water (80 ml), alkalified with sodium hydroxide, warmed and stirred. Thereafter, it was filtered, washed with water and dried to give a dry product, which was recrystallized from ethylene glycol and then from isopropanol to give white crystalline 2-anilino-3-methyl-6-(N-ethyl-N-1'-methylethylamino)fluoran (1.60 g). M.P. 199-200°C. This compound develops black with silica gel and bisphenol-A.

Example 26

In the same manner as in Example 25 but using 2-methyl-4-hydroxy-diphenylamine (1.0 g) in place of 2-methyl-4-methoxy-diphenylamine, the reaction was carried out to give the same fluoran compound (1 g) as in Example 25.

Example 27

In the same manner as in Example 25 but using 2-(4-N-ethyl-N-1'-methylpropylamino-2-hydroxybenzoyl)benzoic acid (1.71 g. M.P. 164.5-165.5°C) in place of 2-(4-N-ethyl-N-1'-methylethylamino-2-hydroxybenzoyl)benzoic acid, the reaction and recrystallization were carried out to give white crystalline 2-anilino-3-methyl-6-(N-ethyl-N-1'-methyl-

propylamino)fluoran (1.60 g). M.P. 169-170°C. This compound develops black with silica gel and bisphenol-A.

Example 28

In the same manner as in Example 25 but using 2-(4-N-methyl-N-1'-methylpropylamino-2-hydroxybenzoyl)-benzoic acid (1.64 g. M.P. 165-166°C) in place of 2-(4-N-ethyl-N-1'-methylethylamino-2-hydroxybenzoyl)benzoic acid, the reaction was carried out. The product was recrystallized from ethylene glycol and then from water-containing methanol to give white crystalline 2-anilino-3-methyl-6-(N-methyl-N-1'-methylpropylamino)fluoran (1.20 g). M.P. 114-117°C. This compound develops black with silica gel and bisphenol-A.

Example 29

In the same manner as in Example 25 but using 2-(4-N-ethyl-N-1'-ethylpropylamino-2-hydroxybenzoyl)benzoic acid (1.78 g. M.P. 175-176°C) in place of 2-(4-N-ethyl-N-1'-methylethylamino-2-hydroxybenzoyl)benzoic acid, the reaction was carried out. The product was recrystallized from 1,2,4-trimethylbenzene and then from isopropanol to give white crystalline 2-anilino-3-methyl-6-(N-ethyl-N-1'-ethylpropylamino)fluoran (1.50 g). M.P. 174.5-175,5°C. This compound develops black with silica gel, bisphenol-A and benzyl 4-hydroxybenzoate.

Examples 30 to 32

In the same manner as in Example 25 but using a benzoylbenzoic acid derivative shown in Table 4, the reaction was carried out to give a fluoran compound as shown in

the same table. All the fluoran compounds produced in these Examples develop black with silica gel and bisphenol-A.

Table 4

| Ex. No. | Benzoylbenzoic acid derivative | Fluoran compound |
|---|---|---|
| 30 | 2-(4-N-ethyl-N-1'-methylbutylamino-2-hydroxy-benzoyl)benzoic acid M.P. 134-135°C | 2-anilino-3-methyl-6-(N-ethyl-N-1'-methylbutylamino)fluoran M.P. 109-112°C |
| 31 | 2-(4-N-ethyl-N-1',2'-dimethylpropylamino-2-hydroxy-benzoyl)-benzoic acid M.P. 172.5-173.5°C | 2-anilino-3-methyl-6-(N-ethyl-N-1',2'-dimethylpropylamino)fluoran M.P. 117-122°C |
| 32 | 2-(4-N-ethyl-N-1',3'-dimethylbutylamino-2-hydroxy-benzoyl)-benzoic acid M.P. 141-142°C | 2-anilino-3-methyl-6-(N-ethyl-N-1',3'-dimethylbutylamino)fluoran M.P. 105-108°C |

Example 33

In the same manner as in Example 26 but using 2-(4-N-methoxyethyl-N-1'-methylethylamino-2-hydroxybenzoyl)-benzoic acid (1.79 g. M.P. 105-108°C) in place of 2-(4-N-methyl-N-1'-methylpropylamino-2-hydroxybenzoyl)benzoic acid, the reaction was carried out to give 2-anilino-3-methyl-6-(N-methoxyethyl-N-1'-methylethylamino)fluoran (1.20 g). M.P. 101-103°C. This compound develops black with silica gel and bisphenol-A.

Example 34

In a solution of 2-(4-N-ethyl-N-1'-methylpropylamino-2-hydroxybenzoyl)benzoic acid (1.71 g. M.P. 164.5-165.5°C) in 96 % sulfuric acid (15 g), N-acetyl-2-methyl-4-methoxy-diphenylamine (1.06 g) was dissolved at a

temperature of from 25 to 30°C and stirred at the same temperature for 24 hours. The resultant solution was poured in ice-water (80 g), and precipitate was filtered and washed with water. The filter cake was taken in water (40 ml) and strongly alkalified with sodium hydroxide. To the mixture, toluene (10 g) was added and stirred at 85°C for 3 hours. An organic layer was separated and washed with warm water (10 ml x 2). From the solution, toluene was distilled off, and isopropanol (10 g) and potassium hydroxide (0.3 g) were added and heated for 2 hours. The solution was cooled to precipitate a crystalline product, which was filtered and washed with isopropanol. The product was further purified with isopropanol to give white crystalline 2-anilino-3-methyl-6-(N-ethyl-N-1'-methylpropylamino)fluoran (1.90 g). M.P. 169-170°C.

Example 35

In a solution of 2-(4-N-1'-isobutyl-3'-methylbutyl- N-methylamino-2-hydroxybenzoyl)benzoic acid (3.98 g. M.P. 171-172°C) in 96 % sulfuric acid (25 g), 2-methyl-4-methoxy-diphenylamine (2.13 g) was dissolved at a temperature of from 10 to 15°C and stirred at the same temperature for 24 hours. The resultant solution was poured in ice-water (100 g), and precipitate was filtered and washed with water. The filter cake was taken in water (50 ml) and stirred together with sodium hydroxide (2.5 g) and toluene (20 g) at 85°C for 3 hours. An organic layer was separated and washed with warm water (30 ml x 2). After distilling toluene off, the residue was mixed with methanol (10 g) and

heated. After cooling, precipitated crystal was filtered and washed with methanol followed by purification with 80 % acetone (10 g) and drying to give white crystalline 2-anilino-3-methyl-6-(N-1'-isobutyl-3'-methylbutyl-N-methylamino)fluoran (4.0 g). M.P. 164-165°C. This fluoran compound develops black with benzyl p-hydroxybenzoate and bisphenol-A.

Example 36

In a solution of 2-(4-N-1'-propylbutyl-N-methylamino-2-hydroxybenzoyl)benzoic acid (3.7 g. M.P. 144.5-146°C) in 96 % sulfuric acid (25 g), 2-methyl-4-methoxydiphenylamine (2.13 g) was dissolved at a temperature of from 10 to 15°C and stirred at the same temperature for 24 hours. The resultant solution was poured in ice-water (80 g), and precipitate was filtered and washed with water. The filter cake was taken in water (50 ml) and stirred together with sodium hydroxide (2.5 g) and toluene (15 g) at 85°C for 2 hours. An organic layer was separated and washed. After distilling toluene off, the residue was mixed with acetone (8 g) and heated. The resultant solution was added with water (2 g) and cooled. Precipitated crystal was filtered and washed with 80 % acetone (15 ml) and dried. Then, it was heated with isopropanol (5 g) and cooled. Precipitated crystal was filtered, washed with isopropanol and dried to give white crystalline 2-anilino-3-methyl-6-(N-1'-propylbutyl-N-methylamino)fluoran (2.85 g). M.P. 146-148°C. This fluoran compound develops black with silica gel and bisphenol-A.

Example 37

2-(4-N-1'-Isobutyl-3'-methylbutyl-N-methylamino-2-hydroxybenzoyl)benzoic acid (1.99 g) and 2,4'-dimethyl-4-hydroxy-diphenylamine (1.07 g) were dissolved in 96 % sulfuric acid (15 g) and stirred at a temperature of from 10 to 15°C for 24 hours. The resultant solution was poured in ice-water (80 g), and precipitate was filtered and washed with water. The filter cake was taken in water, alkalified with sodium hydroxide, heated, filtered, washed with water and dried. The residue was purified with 90 % methanol and isopropanol and dried to give white crystalline 2-(4'-methylanilino)-3-methyl-6-(N-1'-isobutyl-3'-methylbutyl-N-methylamino)fluoran (1.55 g). M.P. 176-178°C. This fluoran compound develops black with silica gel and greenish black with bisphenol-A.

Example 38

In the same manner as in Example 37 but using 2-(4-N-1'-propylbutyl-N-methylamino-2-hydroxybenzoyl)benzoic acid (1.85 g) in place of 2-(4-N-1'-isobutyl-3'-methylbutyl-N-methylamino-2-hydroxybenzoyl)benzoic acid, the reaction was carried out to give white crystalline 2-(4'-methylanilino)-3-methyl-6-(N-1'-propylbutyl-N-methylamino)fluoran (1.5 g). M.P. 173-174°C. This fluoran compound develops black with silica gel and greenish black with bisphenol-A.

Example 39

A mixture of 96 % sulfuric acid (15 g), 2-(4-N-1'-propylbutyl-N-methylamino-2-hydroxybenzoyl)benzoic acid (1.85 g) and 4-methoxy-diphenylamine (1.99 g) was stirred at

a temperature of from 10 to 15°C for 24 hours. The resultant solution was poured in ice-water (80 g), and precipitate was filtered and washed with water. The filter cake was taken in water, alkalified with sodium carbonate, filtered, washed with water and dried. The residue was heated with ethylene glycol (5 g) at 165°C for 5 minutes. After cooling, the mixture was diluted with methanol (5 g), filtered and washed with methanol. Filtered product was purified with isopropanol to give white crystalline 2-anilino-6-(N-1'-propylbutyl-N-methylamino)fluoran (1.3 g). M.P. 163-164°C. This fluoran compound develops dark green with silica gel and bisphenol-A.

Example 40

A mixture of 96 % sulfuric acid (15 g), 2-(4-N-1'-isobutyl-3'-methylbutyl-N-methylamino-2-hydroxybenzoyl)-benzoic acid (1.99 g) and 2'-chloro-4-methoxy-diphenylamine (1.17 g) was stirred to react at a temperature of from 10 to 15°C for 24 hours. The resultant solution was poured in ice-water, and precipitate was filtered and washed with water. The filter cake was taken in water (50 ml) and stirred together with sodium hydroxide (2.5 g) and toluene (10 g) at 85°C for 2 hours. An organic layer was separated and washed with water. After distilling toluene off, the residue was repeatedly recrystallized from 90 % isopropanol to give white crystalline 2-(2'-chloroanilino)-6-(N-1'-isobutyl-3'-methylbutyl-N-methylamino)fluoran (1.6 g). M.P. 90-92°C. This fluoran compound develops purplish black with silica gel and black with bisphenol-A.

0176161

Example 41

In the same manner as in Example 40 but using 2-(4-N-1'-propylbutyl-N-methylamino-2-hydroxybenzoyl)benzoic acid (1.85 g) in place of 2-(4-N-1'-isobutyl-3'-methylbutyl-N-methylamino-2-hydroxybenzoyl)benzoic acid, the reaction was carried out. The crude product obtained after distillation of toluene was recrystallized from isopropanol and 90 % isopropanol to give 2-(2'-chloroanilino)-6-(N-1'-propylbutyl- N-methylamino)fluoran (2.0 g). M.P. 80-82°C. This fluoran compound develops purplish black with silica gel and black with bisphenol-A.

Example 42

In the same manner as in Example 40 but using 2-chloro-4-methoxy-diphenylamine (1.17 g) in place of 2'-chloro-4-methoxy-diphenylamine, the reaction was carried out. The crude product obtained after distillation of toluene was repeatedly purified from 80 % methanol to give white crystalline 2-anilino-3-chloro-6-(N-1'-isobutyl-3'-methylbutyl-N-methylamino)fluoran (1.5 g). M.P. 90-92°C. This fluoran compound develops black with silica gel and bisphenol-A.

Example 43

A mixture of 2-anilino-3-methyl-6-(N-ethyl-N-1'-methylpropylamino)fluoran (5 parts), a 10 % aqueous solution of hydroxyethylcellulose (5 parts) and water (15 parts) was dispersed by means of glass beads to obtain a dispersion in which particle size of the dispersoid was 2 to 3 microns (Dispersion A). Bisphenol-A (10 parts), a 10 % aqueous

solution of hydroxyethylcellulose (10 parts) and water (30 parts) was dispersed by means of glass beads to obtain a dispersion in which particle size of the dispersoid was 2 to 3 microns (Dispersion B).

On the other hand, a mixture of powdery calcium carbonate (20 parts), a 5 % aqueous solution of methylcellulose (20 parts) and water (60 parts) was dispersed to obtain a dispersion (Dispersion C).

Dispersion A (10 parts), Dispersion B (20 parts) and Dispersion C (30 parts) were mixed. To the mixture, a 20 % aqueous solution of a salt of isobutylene/maleic acid copolymer (10 parts) was added and stirred.

The resultant mixture was coated on a sheet of wood free paper (50g/m²) with a wire bar so that an amount of the coated material was 5 g/m² after drying and dried with hot air at 60°C for 1 (one) minute.

The thus produced heat sensitive paper was printed by means of a thermal gradient tester at 150°C under a load of 2 kg with contact time of 1 (one) second to develop a deep black image. When it was printed by means of a printer of a high-speed facsimile equipment, its coloring sensitivity was excellent. The developed black image and the undeveloped surface of the recording paper showed extremely good resistance against light, water and a plasticizer.

When, as a developer, benzyl p-hydroxybenzoate was used in place of bisphenol-A, substantially the same results were achieved.

<u>Example 44</u>

A solution of 2-anilino-3-methyl-6-(N-ethyl-N-1'-ethylpropylamino)fluoran (5 parts) in isopropylnaphthalene (100 parts) was added to a solution of gelatin (25 parts) and gum arabic (25 parts) in water (350 parts) kept at 50°C and emulsified. To the thus prepared emulsion, warm water (1,000 parts) was added and stirred at 50°C for 30 minutes, and then 10 % aqueous solution of sodium hydroxide (1 part) was added and stirred at 50°C for 30 minutes. After adjusting pH of the emulsion to 4.5 by the addition of a dilute aqueous solution of acetic acid, the emulsion was stirred at 50°C for 1 (one) hour and at 5°C for 30 minutes. In this emulsion, the encapsulating material was cured by gradual addition of a 5 % aqueous solution of glutaraldehyde (40 parts). After adjusting pH of the emulsion to 6 with a dilute aqueous solution of sodium hydroxide, it was stirred for several hours at a room temperature. To the thus produced emulsion of capsules, cellulose fine powder (30 parts) and a 10 % aqueous solution of oxidized starch (100 parts) were added. The resultant mixture was coated on a sheet of base paper (45g/m²) so that an amount of the coated material was 5 g/m² after drying and dried to obtain an upper sheet of a pressure sensitive recording paper.

The thus produced upper sheet was laminated on a lower sheet on which, as a developer, a formalin-nobolac resin of phenol and alkylphenol or a mixture of activated clay treated with an acid and zinc salt of 3,5-bis(alpha-methylbenzyl)salicylic acid was coated to form a pressure sensitive recording paper. When the recording paper was

pressed with a pencil or a type of a typewriter, it developed black.

The pressure sensitive recording paper had good shelf stability and high color-developing rate. The developed image had good light resistance for a long time and also good resistance against water and a plasticizer.

What is claimed is:

1. A fluoran compound of the formula:

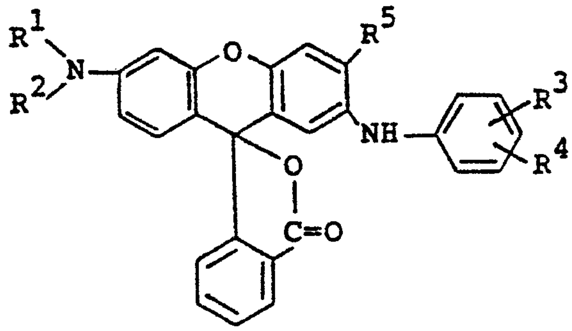

(I)

wherein $R^1$ is $C_3$-$C_{13}$ secondary alkyl with respect to the carbon atom bonded to the nitrogen atom, $R^2$ is $C_1$-$C_4$ primary alkyl, $C_3$-$C_4$ alkenyl or $C_3$-$C_4$ alkoxyalkyl, $R^3$ and $R^4$ are, same or different, hydrogen, halogen, $C_1$-$C_4$ alkyl or trifluoromethyl, $R^5$ is hydrogen, methyl, ethyl or halogen.

2. A fluoran compound according to claim 1, wherein $R^1$ is 1'-methylpropyl, $R^2$ is ethyl, $R^3$ and $R^4$ are hydrogen, and $R^5$ is methyl.

3. A fluoran compound according to claim 1, wherein $R^1$ is 1'-ethylpropyl, $R^2$ is ethyl, $R^3$ and $R^4$ are hydrogen, and $R^5$ is methyl.

4. A fluoran compound according to claim 1, wherein $R^1$ is 1'-methylethyl, $R^2$ is butyl, $R^3$ and $R^4$ are hydrogen, and $R^5$ is methyl.

5. A fluoran compound according to claim 1, wherein $R^1$ is 1'-methylpropyl, $R^2$ is butyl, $R^3$ and $R^4$ are hydrogen, and $R^5$ is methyl.

6. A fluoran compound according to claim 1, wherein $R^1$ is 1'-methylpropyl, $R^2$ is ethyl, $R^3$ is hydrogen, $R^4$ is chloro which is at a para-position with respect to the amino group, and $R^5$ is methyl.

7. A fluoran compound according to claim 1, wherein $R^1$ is 1'-ethylpropyl, $R^2$ is ethyl, $R^3$ is hydrogen, $R^4$ is chloro which is at a para-position with respect to the amino group, and $R^5$ is methyl.

8. A fluoran compound according to claim 1, wherein $R^1$ is 1'-propylbutyl, $R^2$ is methyl, $R^3$ and $R^4$ are hydrogen, and $R^5$ is methyl.

9. A fluoran compound according to claim 1, wherein $R^1$ is 1'-isobutyl-3'-methylbutyl, $R^2$ is methyl, $R^3$ amd $R^4$ are hydrogen, and $R^5$ is methyl.

10. A method for preparing a fluoran compound (I) according to claim 1 comprising reacting a benzoylbenzoic acid derivative of the formula:

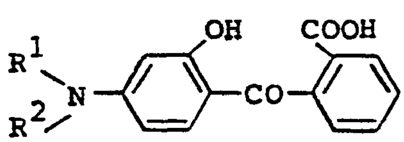

(II)

wherein $R^1$ and $R^2$ are the same as defined above with a hydroxydiphenylamine derivative of the formula:

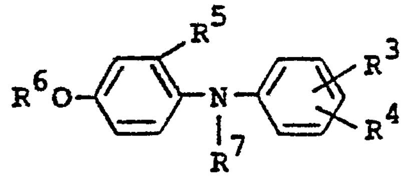

(III)

wherein $R^3$, $R^4$ and $R^5$ are the same as defined above, $R^6$ is hydrogen or $C_1$-$C_4$ alkyl and $R^7$ is hydrogen or a group of the formula: $-COR^6$.

11. A method according to claim 10, wherein $R^7$ is the group of the formula: $-COR^6$, and the method further comprises deacylating said group.

12. A method according to claim 10, wherein the reaction is carried out in the presence of a condensation agent.

13. A method according to claim 10, wherein a reaction temperature is from -5 to 90°C.

14. A benzoylbenzoic acid derivative of the formula:

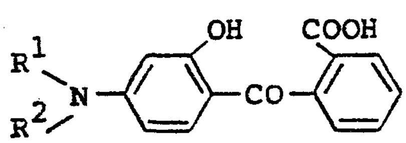

(II)

wherein $R^1$ and $R^2$ are the same as defined above.

15. A method for preparing a benzoilbenzoic acid derivative (IV) accoring to claim 14 comprising reacting an N-disubstituted m-aminophenol compound of the formula:

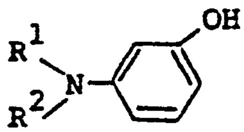

(IV)

wherein $R^1$ and $R^2$ are the same as defined above with phthalic anhydride.

16. A method according to claim 15 wherein a molar ratio of the compound (IV) and phthalic anhydride is from 1:1 to 1:2.

17. An N-disubstituted m-aminophenol compound of the formula:

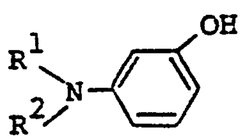

(IV)

wherein $R^1$ and $R^2$ are the same as defined above.

18. A method for producing an N-disubstituted m-aminophenol compound (IV) according to claim 17 comprising reacting an N-monoalkyl-m-aminophenol compound of the formula:

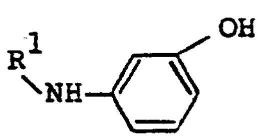

(V)

wherein $R^1$ is the same as defined above with an alkylating agent of the formula:

$R^2$-Y (VI)

wherein $R^2$ is the same as defined above and Y is a residue of the alkylating agent.

19. An N-monoalkyl-m-aminophenol compound of the formula:

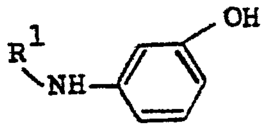

(V)

wherein $R^1$ is the same as defined above.

20. A method for preparing an N-monoalkyl-m-aminophenol compound (V) according to claim 19 comprising subjecting m-aminophenol to reductive alkylation with a ketone compound of the formula:

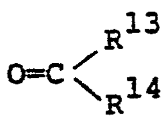

(VII)

wherein $R^{13}$ and $R^{14}$ are $R^{11}$ and $R^{12}$, respectively or groups which are converted to $R^{11}$ and $R^{12}$, respectively during the reaction, and $R^{11}$ and $R^{12}$ are, same or different, straight or branched $C_1$-$C_{11}$ alkyl provided that the total number of the carbon atoms of $R^{11}$ and $R^{12}$ is 2 to 12.

21. A pressure or heat sensitive recording paper comprising supporting paper and at least one fluoran compound (I) according to claim 1 and a developer applied on the paper.

European Patent Office

# EUROPEAN SEARCH REPORT

0176161
Application number

EP 85301195.5

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| P,X | PATENT ABSTRACTS OF JAPAN, unexamined application, C field, vol. 9, no. 175, July 19, 1985<br>THE PATENT OFFICE JAPANESE GOVERNMENT<br>page 130 C 292<br>* Kokai-No. 60-47 068 (TAOKA KAGAKU KOGYO K.K.) *<br>-- | 1-14 | C 07 D 493/10<br>C 07 C 101/78<br>C 07 C 99/00<br>C 07 C 91/44<br>C 07 C 89/00<br>B 41 M 5/12<br>(C 07 D 493/10<br>C 07 D 311:00<br>C 07 D 307:00) |
| X | EP - A1 - 0 081 228 (JUJO PAPER CO., LTD.)<br>* Claims 1,2,5; page 6, lines 9,10 *<br>-- | 1-9,21 | |
| X | BEILSTEINS HANDBUCH DER ORGANISCHEN CHEMIE, 4th edition, E III, vol. 13, part 2, 1973<br>Springer-Verlag, Berlin-Heidelberg-New York<br>* Page 943 *<br>-- | 19 | **TECHNICAL FIELDS SEARCHED (Int Cl 4)** |
| | AT - B - 327 237 (CIBA-GEIBY AG) | | C 07 D 493/00<br>C 07 C 101/00<br>C 07 C 99/00<br>C 07 C 91/00<br>C 07 C 89/00<br>B 41 M 5/00 |
| X | * Formula (4); page 3, lines 22-24 * | 14-17 | |
| A | * Page 1, lines 1-3; page 3, lines 11-15; formula 6 *<br>-- | 1,10,19,21 | |

The present search report has been drawn up for all claims

| Place of search | Date of completion of the search | Examiner |
|---|---|---|
| VIENNA | 29-11-1985 | BRUS |

**CATEGORY OF CITED DOCUMENTS**

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

European Patent Office

# EUROPEAN SEARCH REPORT

Application number 0176161



EP 85301195.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl 4) |
|---|---|---|---|
| X | PATENT ABSTRACTS OF JAPAN, unexamined application, C field, vol. 6, no. 266, December 25, 1982<br>THE PATENT OFFICE JAPANESE GOVERNMENT<br>page 51 C 142<br>* Kokai-No. 57-156 446 (SUMITOMO KAGAKU KOGYO K.K.) *<br>-- | 20 | |
| A | US - A - 4 330 473 (HATANO et al.)<br>* Abstract; column 4, formula II*<br>-- | 1-14,21 | |
| A | AT - B - 322 578 (YAMAMOTO KAGAKU GOSEI KABUSHIKI KAISHA)<br>* Claim 1; page 3, formula V *<br>-- | 1-14,21 | TECHNICAL FIELDS SEARCHED (Int. Cl 4) |
| A | AT - B - 315 825 (THE NATIONAL CASH REGISTER COMPANY)<br>* Claim 1 *<br>---- | 1-14,21 | |

The present search report has been drawn up for all claims

| Place of search | Date of completion of the search | Examiner |
|---|---|---|
| VIENNA | 29-11-1985 | BRUS |

CATEGORY OF CITED DOCUMENTS

X particularly relevant if taken alone
Y particularly relevant if combined with another document of the same category
A technological background
O non-written disclosure
P intermediate document
T theory or principle underlying the invention
E : earlier patent document, but published on, or after the filing date
D : document cited in the application
L : document cited for other reasons
& : member of the same patent family, corresponding document

EPO Form 1503 03 82